# EUROPÄISCHE PATENTSCHRIFT

(11) **EP 0 433 426 B1**
(45) Veröffentlichungstag und Bekanntmachung des Hinweises auf die Patenterteilung: **02.02.1994**
(21) Anmeldenummer: 90910672.6
(22) Anmeldetag: 04.07.1990
(51) Int. Cl.: F16M 11/04

(54) **MIT ZUSATZVORRICHTUNGEN AUSGESTATTETES STATIV FÜR DIE HALTERUNG EINES FREI POSITIONIERBAREN GERÄTES**
STAND EQUIPPED WITH ACCESSORY DEVICES, FOR SUPPORTING A FREELY ORIENTABLE APPARATUS
STATIF EQUIPE DE DISPOSITIFS ACCESSOIRES, POUR SUPPORTER UN APPAREIL LIBREMENT ORIENTABLE

(30) Priorität: 04.07.1989 DE 3921857
(43) Veröffentlichungstag der Anmeldung: 26.06.1991
(73) Patentinhaber: Leica AG, CH-9435 Heerbrugg (CH)
(72) Erfinder: MÜNTENER, Jürg, CH-9436 Balgach (CH); GESCHWENTNER, Otto, CH-9436 Balgach (CH); METZ, Jürgen, CH-9436 Balgach (CH)
(86) Internationale Anmeldenummer: EP9001075
(87) Internationale Veröffentlichungsnummer: WO9100472

(56) Entgegenhaltungen:
- EP-A- 0 023 003
- EP-A- 0 023 004
- EP-A- 0 048 404
- EP-A- 0 049 261
- DE-A- 3 617 751
- DE-A- 3 728 527
- DE-U- 8 909 957
- GB-A- 2 074 337
- GB-A- 2 176 764

## Beschreibung

Die Erfindung betrifft Zusatzvorrichtungen an einem Stativ für Geräte, insbesondere Operationsmikroskope, die in einem vorbestimmten Raum frei beweglich und bezüglich ihrer Lage und ihrer Zuordnung zu einem Objekt in diesem Raum frei positionierbar sind. Derartige Anforderungen werden speziell an Operationsmikroskope gestellt, die in der HNO-Chirurgie eingesetzt werden; sie kommen aber auch im Bereich der Neuro- und der Plastischen Chirurgie im vermehrten Maße zum Einsatz. In Abhängigkeit vom jeweiligen Einsatzgebiet und der gewählten Aufgabenstellung können an das eigentliche Operationsmikroskop diverse Zusatz-Module, wie ein Mitbeobachtungs-Tubus, ein Fotostutzen, eine Kamera u.s.w., in bekannter Weise angeflanscht werden. Dabei vergrößern sich die ohnehin schon vorhandenen Austarierungs-Probleme für das eigentliche Beobachtungsgerät (Mikroskop).

Aus der EP-A-23003 ist eine Tragvorrichtung für ein optisches Beobachtungsgerät bekannt, bei dem mehrere als Gelenkparallelogramme ausgebildete Schwenksysteme die Beweglichkeit des Mikroskops erlauben. Dabei kommt ein verstellbares Gegengewicht für die freie Positionierung im Raum zum Einsatz. Außerdem sichern elektromagnetische Bremslager die Fixierung der optimalen Stativ-Einstellung.

In der EP-A-23004 wird eine Zusatzvorrichtung beschrieben, die insbesondere in der Lage ist, Dreh- und Kippmomente zu kompensieren, indem die Schwerpunktlage des Mikroskops mit seinem Zubehör bestimmt und das Gleichgewicht danach eingestellt werden kann.

In der EP-A-49251 ist eine Stütze für ein optisches Gerät dargestellt, das ein Gestängesystem als Trägereinrichtung aufweist, welches sich durch ein Kugellager erstreckt. An dem einen (unteren) Ende ist das Gerät befestigt, an dem anderen (oberen) Ende ist ein Gegengewicht in der Form befestigt, daß es um drei senkrecht zueinander stehende Achsen verschiebbar ist. Dieses verstellbare Gegengewicht erlaubt eine freie Positionierung des Mikroskops im Raum.

Aus der DE-OS 36 17 751 ist eine Mikroskopeinheit bekannt, bei der ein einstellbares Gegengewicht zum Einsatz kommt, wodurch ein Ausgleich des Momentes ermöglicht wird, welches um Schwenk- und Kippachsen entsteht und welches aus der Hinzufügung oder Entfernung von Bauelementen oder Modulen resultiert.

Darüber hinaus ist aus dem Wild-Firmenprospekt (Bedienungsanleitung) M2-690-XI.86 vom November 1986 ein Operationsmikroskop-Stativ bekannt, bei dem das Feindispositionieren, also das Schwenken und Neigen des Mikroskops mit Hilfe von Triebknöpfen und Neigehebeln an Triebgelenken möglich ist.

Weiterhin ist es aus dem Zeiss-Prospekt W 30-082-d vom Mai 1988 ("Bodenstativ S 21") bekannt, bei einem aus Tragarm und Federarm bestehenden Gelenkarm eine von außen zugängliche Gewichtseinstellung für die Mikroskopausrüstung sowie Bremsen für eine individuelle Einstellung der Gängigkeit der Gelenke und der Auf/Abbewegung vorzusehen.

Schließlich ist aus der EP-A-48 404 ein verstellbares Stativ für optische Beobachtungsgeräte bekannt, das einen Stativfuß, eine Stativsäule und einen als Gelenkparallelogramm ausgebildeten Tragarm aufweist, wobei zum Ausgleich des Gewichtes des verwendeten Beobachtungsgerätes im Parallelogramm eine verstellbare Feder angeordnet ist.

Dem geschilderten Stand der Technik haften einzeln oder in Kombination folgende Nachteile an. Ein wesentlicher Nachteil aller bekannten Lösungen besteht darin, daß die Schwergängigkeit der Schwenkarme, auch selbst dann, wenn sie einstellbar ist, einen sehr hohen Kraftaufwand für die Auf- bzw. Abbewegung des Mikroskops erfordert. Die Folge diese hohen Kraftaufwandes ist, daß eine sehr genaue Positionierung des Mikroskops in der Höhe ohne die zusätzliche Bedienung eines Fokussiertriebes nicht möglich ist. Diese zusätzliche Bedienung wiederum bedeutet eine Unterbrechung der unter dem Mikroskop auszuführenden - beispielsweise mikrochirurgischen - Arbeit. Es ist ohne weiteres einsichtig, daß Verzögerungen bzw. Unterbrechungen dieser Art in hohem Maße stören bzw. gefährlich sind. Ein weiterer, wesentlicher Nachteil dieses hohen Kraftaufwandes ist, daß es beim Operateur, der selber sehr feine Arbeiten durchführt, wegen des von ihm auszuübenden Kraftaufwandes zu Muskelverspannungen kommen kann, die den Fortgang der Mikro-Operation hemmen bzw. nachteilig beeinflussen. Ganz allgemein kann gesagt werden, daß es eine Unterbrechung des Operations-Geschehens bedeutet, wenn zum Zwecke einer Positionsänderung des optischen Gerätes die Operation unterbrochen werden muß, weil etwa die Triebknöpfe bedient werden müssen.

Nachteile der genannten EP-A-23003 ergeben sich aus dem notwendigen Aufwand, der beim Auswechseln eines Gerätes gegen ein anderes mit einem anderen Gewicht entsteht. Dieser Aufwand wird noch dadurch erschwert, daß die Auffindung der Schwerpunktlage und das Einstellen des Gleichgewichtes nicht logisch leicht nachvollziehbar und nicht ohne weiteres durchführbar ist. Komplizierte, zeitaufwendige Gleichgewichtseinstellungen, die zudem auch für das Operationspersonal nicht leicht nachvollziehbar sind, wirken einer in der modernen Operationsmikroskopie heute zu fordernden hohen Zubehörflexibilität entgegen.

Es ist daher die Aufgabe der vorliegenden Erfindung, Zusatzvorrichtungen für ein Stativ zur Aufnahme beispielsweise eines Mikroskops anzugeben, wobei dieses Stativ eine leichtgängige Positionierung des Gerätes vor allem in der Höhe gestattet. Dabei soll zusätzlich sichergestellt sein, daß jedwede Positionsänderung des Mikroskops während der Operation zu keiner störenden Unterbrecnung derselben führt. Außerdem besteht die Aufgabe darin, die bei jedem Wechsel der verschiedenartigsten Zubehör-Baugruppen für das Mikroskop sich ergebenden Neueinstellungen des Gleichgewichts des Gesamtsystems in sich logisch und schnell durchführbar zu gestalten. Schließlich besteht die Aufgabe der vorliegenden Erfindung darin, die Austarierung des Gerätes (Mikroskops) mitsamt seinem Zubehör ohne Gegengewichts-Verschiebung, also ohne aufwendige Bestimmung der Schwerpunktlage des Mikroskops mitsamt seinem Zubehör, zu realisieren.

Diese Aufgabe wird durch den Gegenstand des Anspruchs 1 gelöst. Weitere vorteilhafte Ausgestaltungen finden sich in den Unteransprüchen 2 bis 10.

In den Zeichnungen sind Ausführungsbeispiele der vorliegenden Erfindung schematisch dargestellt. Es zeigen:
- Fig. 1a:: einen Teil einer Stativaufhängung mit angesetztem Operationsmikroskop in Seitendarstellung;
- Fig. 1b:: das in Fig. 1a Dargestellte in Draufsicht;
- Fig. 2:: die Darstellung der Fig. 1 in Vorderansicht;
- Fig. 3:: ein Bodenstativ mit einem Gelenkparallelogramm-Gestänge und erfindungsgemäßer Zusatzeinrichtung;
- Fig. 4:: eine Detailansicht des Gelenkparallelogramm-Gestänges mit einer anderen erfindungsgemäßen Zusatzeinrichtung;
- Fig. 5a - 5c:: schematisierte Detail-Ansichten von Fig. 4 betreffend die unterschiedlichen Stellungen eines Bauteils in Abhängigkeit von der Hubhöhe des (optischen) Gerätes.

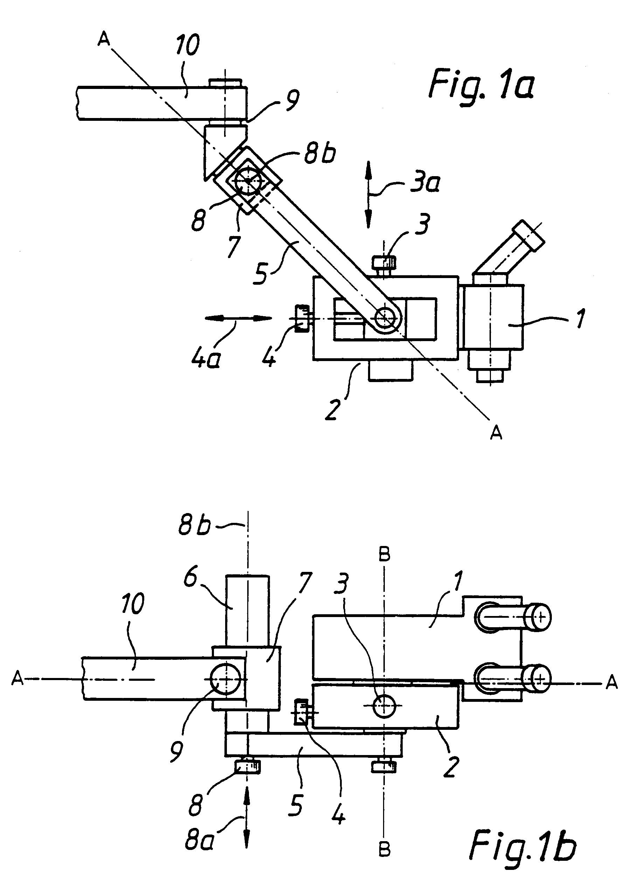

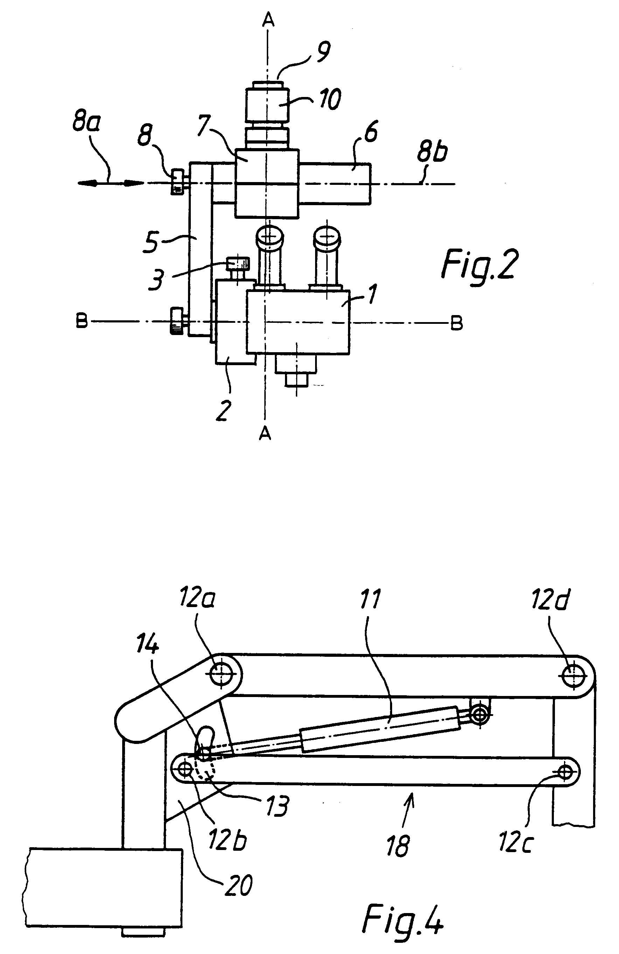

In Fig. 1a ist ein Gerät, beispielsweise ein Operationsmikroskop, mit der Bezugsziffer 1 dargestellt, das an einer Tragvorrichtung 2 adaptiert ist, welche an dem unteren Ende eines Tragarms 5 angelenkt ist. Der Tragarm 5 ist in seinem oberen Teil mit einem Schlitten 6 verbunden, der in einer Führung 7 entlang der Translationsachse 8b, also in Richtung des Doppelpfeils 8a, mit Hilfe der Spindel 8 verstellt werden kann. Über ein Drehlager 9 ist die beschriebene Aufhängung mit einem Tragarm 10 rotatorisch verbunden. Die Tragvorrichtung 2 weist zwei Baugruppen auf, die mittels der Verstellvorrichtungen 3 bzw. 4 in Richtung des Doppelpfeils 3a bzw. 4a feinverstellt werden können. Aus der Darstellung der Verstellrichtungen 3a, 4a bzw. 8a in den Fig. 1 und 2 wird ersichtlich, daß das Gerät 1 in drei Raumkoordinaten-Richtungen, die senkrecht aufeinander stehen, gezielt verstellt werden kann. In den Fig. la und lb ist mit A-A eine nichtsenkrechte Achse dargestellt, die parallel zum Tragarm 5 verläuft und senkrecht auf der horizontalen (nichtvertikalen) Achse B-B steht. Der Schnittpunkt beider Achsen A-A, B-B ist in Fig. 2 dargestellt. Die Achse B-B verläuft durch die Gelenkachse am unteren Ende des Tragarms 5, um die das Gerät 1 verkippt werden kann.

Der Gleichgewichtszustand für das Gerät 1 bzw. für das Gesamtsystem wird nun dadurch hergestellt, daß der Schwerpunkt des Gerätes über Verschiebungen längs der drei Raumrichtungen (3a; 4a; 8a) in den Schnittpunkt der beiden nichtsenkrechten Achsen A-A, B-B gebracht wird.

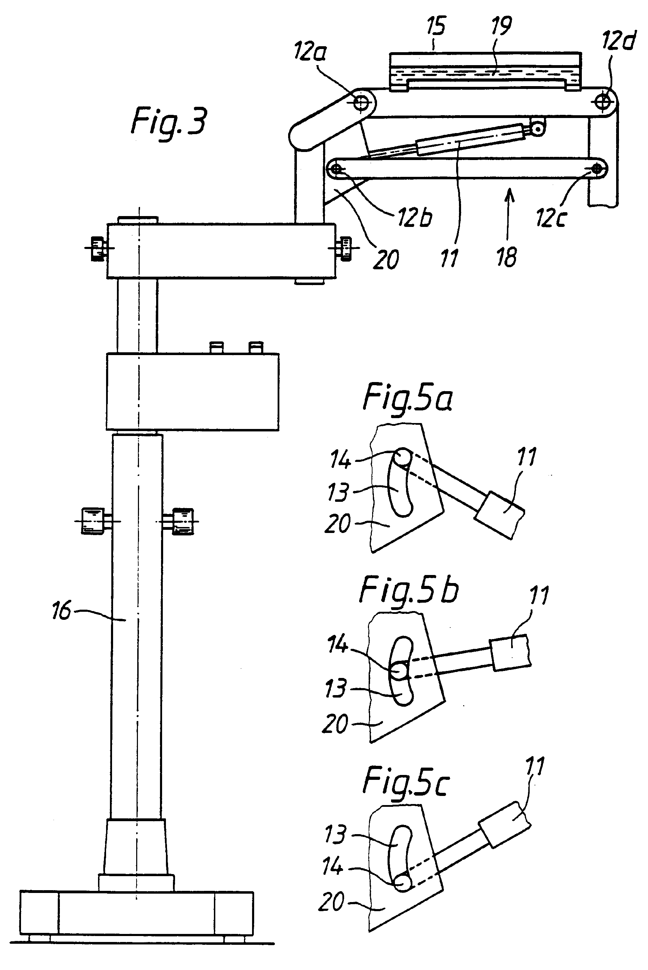

In Fig. 3 befindet sich im oberen Teil eines Bodenstativs 16 ein Gelenkparallelogramm 18, welches mehrere Gestänge aufweist, die über Gelenke 12a - 12d miteinander verbunden sind. Außerdem ist eine Platte 20 dargestellt, die u.a. das Gelenk 12b aufweist. Ein an sich bekanntes Kraftspeicherelement 11 - beispielsweise eine Gasdruckfeder - ist einerseits mit dem oberen Gestänge des Parallelogramms und andererseits mit der Platte 20 verbunden. Dieses Kraftspeicherelement 11 bringt die notwendige Gegenkraft auf, die zum Einstellen eines Gleichgewichtszustandes bei Veränderung der sog. Hubhöhe, also in vertikaler Richtung, erforderlich ist. Um die Eigenschaften des leichten, schnellen und sicheren Positionierens zu ermöglichen, sind an den Gelenken 12a - 12d des Gelenkparallelogramms 18 konstruktive Maßnahmen zur Herabsetzung der Gelenkreibung erforderlich, die eine - in diesem Fall negative - Eigenschaft der Kraftspeicherelemente, nämlich die wechselnde Kraft in Abhängigkeit vom Hub, wirksam werden lassen. Da der Hub des Kraftspeicherelementes 11 abhängig von der Winkelstellung des Gelenkparallelogramms 18 ist, ist eine Kompensation der hubabhängigen Kraftänderung bei herabgesetzter Gelenkreibung notwendig, da sonst das Gleichgewicht am Gelenkparallelogramm mit eingebautem Kraftspeicherelement 11 nur für eine einzige Winkelstellung des Parallelogramms, und damit nur für eine bestimmte Höhe, eingestellt werden kann.

Diese Kompensation der wechselnden Kraft wird durch ein zusätzliches erfindungsgemäßes Bauteil für das Gelenkparallelogramm bewirkt. Dies wird in den Fig. 4 und 5a - 5c näher erläutert. Die erforderliche Kompensation der wechselnden Kraft wird durch eine winkelabhängige Lageveränderung der Aufhängung des Kraftspeicherelementes 11 in der Platte 20 erreicht. Wie aus Fig. 4 ersichtlich, greift die Gasdruckfeder 11 über einen Zapfen 14 in einen Schlitz 13. Der Schlitz ist als kreisbogenförmiges Langloch ausgebildet und stellt die Führung des Zapfens 14 in Abhängigkeit von der Hubhöhe des Gelenkparallelogramms 18 sicher. Die Länge des Schlitzes richtet sich nach den zu kompensierenden Kraftänderungen des Kraftspeicherelementes 11. Der Schlitz bewirkt eine kontinuierliche Führung des Zapfens 14 von einem Ende zum anderen bei der Bewegung des Gelenkparallelogramms 11. In Fig. 5a ist dargestellt, daß bei maximaler Absenkung (Erniedrigung der Hubhöhe) des Gelenkparallelogramms der Zapfen 14 im oberen Anschlag-Bereich des Schlitzes 13 zu liegen kommt, während dieser bei maximaler Hubhöhe - vgl. Fig. 5c - im unteren Anschlag-Bereich des Schlitzes 13 liegt. In Fig. 5b ist die Mittelstellung dargestellt, wie sie sich etwa aus Fig. 4 ergibt (waagerechter Verlauf der beiden Gestänge des Gelenkparallelogramms 18).

Die Funktion dieser erfindungsgemäßen Zusatzvorrichtung ist folgende: Der Schwenkarm oder das Gelenkparallelogramm 18 wird während der Operation beim Einstellen der Höhe des Mikroskops (Hubhöhe) bedient.

Das Kraftspeicherelement 11, hier: die Gasdruckfeder, hat eine Kraft, die in Richtung ihrer Aufhängung in der Platte 20 (also in Richtung zum Zapfen 14 hin) wirkt. Die Länge der Senkrechten zwischen dieser Kraftrichtung und dem Gelenk 12a ist derjenige Hebelarm, der dafür ausschlaggebend ist, ob der Schwenkarm bzw. das Gelenkparallelogramm 18 in der einmal eingestellten Hubhöhe (Schwebe) bleibt oder nicht. Das Gelenkparallelogramm 18 kann in Wirkstellung aus der Waagerechten um ca. 350 nach oben (vg. Fig. 5c) und nach unten (vgl. Fig. 5a) bewegt werden. In der angehobenen Stellung ist die Gasdruckfeder 11 komplett ausgezogen; in der abgesenkten Stellung ist sie komplett eingezogen. In Abhängigkeit dieses Auszugs der Gasdruckfeder 11 ändert sich deren Kraft. In der oberen Stellung des Gelenkparallelogramms 18 ist die Kraft der Gasdruckfeder 11 schwächer; in der unteren Stellung des Gelenkparallelogramms 18 ist die Kraft der Gasdruckfeder 11 stärker. Das bedeutet also, daß sich bei gleichbleibendem Hebelarm die Kraft ändert. Bei den bekannten Schwenkarmen bzw. Gelenkparallelogrammen nach dem Stand der Technik ist diese Änderung durch die Einstellung der Reibgelenke 12a - 12d überdeckt bzw. "vertuscht" worden. Wenn nun aber aus übergeordneten Gründen eines modernen Handlings-Komforts eine Herabsetzung der Gelenkreibung eben dieser Gelenke 12a - 12d vorgenommen werden muß, führt dies automatisch dazu, entfalten: War das Gelenkparallelogramm 18 in einer mittleren Stellung ausbalanciert, so bewirkte die Kraftabnahme der Gasdruckfeder 11 in der oberen Stellung des Gelenkparallelogramms 18 ein Herabfallen und die Kraftzunahme in der unteren Stellung des Gelenkparallelogramms 18 ein eigenständiges Aufsteigen des Gelenkparallelogramms. Daher mußte eine Veränderung des Hebelarms in Abhängigkeit von der Gelenkparallelogramm-Stellung vorgenommen werden. Dies wird mit der in der Platte 20 realisierten kurvenförmigen Schlitzführung für die Aufhängung des Kraftspeicherelementes 11 realisiert. Bei abgesenktem Schwenkarm wandert der Zapfen 14 in der Kurve nach oben, so daß der Hebelarm kürzer wird und die Kraft nicht mehr ausreicht, den Schwenkarm eigenständig nach oben zu drücken. Andererseits folgt bei angehobenem Schwenkarm genau das Gegenteil: der Zapfen 14 wandert nach unten, so daß der Hebelarm größer wird und die Kraft ausreicht, um den Schwenkarm oben zu halten. Obwohl der Schlitz 13 als Teil eines Kreisbogens dargestellt ist, sind prinzipiell auch andere Kurvenführungen möglich.

Anstelle dieser geschilderten erfindungsgemäßen Maßnahme kann bei einer weiteren Ausführungsform der vorliegenden Erfindung eine Kompensation der wechselnden Kraft dadurch erreicht werden, daß die durch das Kraftspeicherelement 11 auszugleichende Gewichtskraft kontinuierlich bei der Bewegung des Gelenkparallelogramms 18 zunimmt, und zwar in der Form, daß diese Zunahme der zunehmenden Kraft des Kraftspeicherelementes 11 entgegenwirkt. Hierzu wird auf Fig. 3 verwiesen, in der das Gelenkparallelogramm 18 mit herkömmlich aufgehängtem Kraftspeicherelement 11 dargestellt ist. Das dargestellte Kraftspeicherelement 11 ist so in das Gelenkparallelogramm 18 eingebaut, daß bei einem abwärts weisenden Parallelogramm die hubabhängige Kraft zunimmt. Zum Ausgleich hierfür kann ein Flüssigkeitsbehälter 15 auf ein Gestänge des Gelenkparallelogramms 18 - vorzugsweise auf das obere - aufgesetzt werden. Die notwendige Gewichtskraftzunahme bei abwärts weisendem Parallelogramm wird sodann in einfacher Weise durch Verlagerung der Flüssigkeit in dem nicht ganz gefüllten Flüssigkeitsbehälter 15 realisiert. Es liegt im Rahmen dieses Erfindungsvorschlages, entweder Behälter unterschiedlicher Raumform bzw. unterschiedlichen Füllungsgrades bzw. unterschiedlicher Behälterflüssigkeit vorzusehen, um alle erforderlichen Kompensations-(Gegen)Kräfte realisieren zu können. Natürlich ist es auch möglich, den Flüssigkeitsbehälter 15 mit einem internen Fluid-Regelkreissystem zur steuerbaren Zu- und Abfuhr von in einem separaten Reservoir befindlichen Flüssigkeit zu verbinden.

Mit dem vorgeschlagenen erfindungsgemäßen Lösungen ist ein wenig kraftaufwendiges sowie ein sehr genaues Positionieren des Mikroskops in der Höhe über dem Operationsfeld ermöglicht worden. Dies führt zu einer stärkeren Konzentration des beispielsweise eine Mikro-Operation ausführenden Chirurgen auf dessen eigentliche Hauptaufgabe und damit zu einer Verkürzung des gesamten Manipulierens in vivo.

### Bezugszeichen-Liste

- 1: - Gerät (beispielsweise Operationsmikroskop)
- 2: - Tragvorrichtung
- 3; 4: - Verstellvorrichtungen (Spindeltriebe)
- 3a; 3b: - Doppelpfeile (Translationsrichtungsanzeiger)
- 5: - Tragarm
- 6: - Schlitten
- 7: - Führung
- 8: - Spindel
- 8a: - Doppelpfeil (Translationsrichtungsanzeiger)
- 8b: - Translationsachse von (6) und (7)
- 9: - Drehlager
- 10: - Tragarm
- 11: - Kraftspeicherelement (Gasdruckfeder)
- 12a,12b,12c,12d: - Gelenke
- 13: - Schlitz
- 14: - Zapfen
- 15: - Flüssigkeitsbehälter
- 16: - Bodenstativ
- 18: - Gelenkparallelogramm
- 19: - Flüssigkeit
- 20: - Platte
- A-A: - (erste) nicht-vertikale Achse
- B-B: - (zweite) nicht-vertikale Achse

## Patentansprüche

1. Stativ für die Halterung eines bezüglich mindestens einer Bezugsebene frei positionierbaren Gerätes - vorzugsweise eines optischen Beobachtungs- und/oder Registriergerätes -, enthaltend einenTragarm (10), ein Drehlager (9) sowie einen Schwenkarm bzw. ein Gelenkparallelogramm (18) mit zugeordnetem Kraftspeicherelement (11), **dadurch gekennzeichnet,**
daß - ein Tragarm (5) über das Drehlager (9) mit dem Tragarm (10) verbunden und senkrecht zur Achse des Drehlagers (9) verschieblich gelagert ist und der Schwenkarm bzw. das Gelenkparallelogramm (18) mit dem Tragarm (10) verbunden ist, und daß das Stativ (16) zur Austarierung und zur reproduzierbaren Feinpositionierung des Gerätes, beispielsweise eines Operationsmikroskops (1), in dessen jeweilige Wirkstellung Mittel zum Verschieben des Schwerpunktes des an eine Tragvorrichtung (2) ansetzbaren Gerätes (1) inklusive eventueller Zusatz-Module in den Schnittpunkt einer parallel zum Tragarm (5) verlaufenden nichtsenkrechten Achse (A-A) mit einer nicht-senkrechten Achse (B-B), die durch eine Gelenkachse am unteren Ende des Tragarms (5) verläuft, um die das Gerät (1) kippbar ist, aufweist, wobei
- die Achsen (A-A) und (B-B) aufeinander senkrecht stehen und
- Mittel zum Ausgleich der vom Hub abhängigen wechselnden Kraft des Kraftspeicherelements (11) vorgesehen sind.

2. Stativ nach Anspruch 1, **dadurch** **gekennzeichnet**,
daß diese Mittel folgende Bauteile umfassen:
(a) eine aus mindestens zwei Baugruppen bestehende Tragvorrichtung (2), an der einerseits beispielsweise das Mikroskop (1) befestigt ist und die andererseits mit einem Tragarm (5) gelenkig verbunden ist, wobei die Baugruppen zur Einstellung eines stabilen Gleichgewichtszustandes des angeflanschten Mikroskops (1) im Raum mittels je einer Verstellvorrichtung (3 bzw. 4) in zueinander senkrecht verlaufender Richtung gezielt verschiebbar sind,
(b) eine Führungsvorrichtung vorzugsweise im oberen Bereich des Tragarms (5), bestehend aus einem in einer Führung (7) mittels einer Spindel (8) entlang einer Translationsachse (8b) - und damit senkrecht zu den Translationsrichtungen, welche durch die Verstellvorrichtungen (3 bzw. 4) vorgegeben sind, - verschiebbar angeordneten Schlitten (6), und
(c) eine Vorrichtung (13,14,20) zur ortsvariablen Aufhängung bzw. Führung des mit dem Glenkparallelogramm (18) zusammenwirkenden Kraftspeicherelementes (11) zur Kompensierung der hub- bzw. winkelabhängigen Änderung der Kraft des Kraftspeicherelementes (11) zwecks permanenter Beibehaltung seines Gleichgewichtes in jeder Höhe des adaptierten Gerätes (1).

3. Stativ nach Anspruch 1 oder 2, **dadurch gekennzeichnet,** daß die Verstellvorrichtungen für die Baugruppen je einen Spindeltrieb (3;4) umfassen und daß die Verstellrichtungen (3a;4a) senkrecht zueinander sowie in einer Ebene verlaufen.

4. Stativ nach mindestens einem der vorhergehenden Ansprüche, **dadurch gekennzeichnet**, daß die Verstellvorrichtungen für die Baugruppen bzw. für den Schlitten (6) längs nicht-linearer - vorzugsweise sphärischer - Bahnen verlaufen.

5. Stativ nach Anspruch 2, **dadurch gekennzeichnet,** daß die Vorrichtung für die ortsvariable Halterung des Kraftspeicherelements (11) einen als kreisbogenförmiges Langloch ausgebildeten Schlitz (13) in einer Platte (20) umfaßt, in welchem ein mit dem Kraftspeicherelement (11), beispielsweise einer Gasdruckfeder, starr verbundener Zapfen (14) als lagernde Achse spielfrei geführt wird.

6. Stativ nach Anspruch 5, **dadurch gekennzeichnet,** daß der Schlitz (13) eine von der Kontur eines Kreises abweichende - vorzugsweise jedoch eine nichtlineare - Form aufweist.

7. Stativ nach mindestens einem der vorhergehenden Ansprüche, **dadurch** **gekennzeichnet**, daß die Gelenke des Parallelogramms (18) Mittel zur gezielt einstellbaren Herabsetzung der Gelenkreibung aufweisen.

8. Stativ nach den Ansprüchen 1 und 2, **dadurch ge****kennzeichnet**, daß anstelle der ortsvariablen Aufhängung zur Kompensation der hubabhängigen Kraftänderung des Kraftspeicherelementes (11) ein mit einer Flüssigkeit (19) teilweise gefüllter Behälter (15) derart dem Gelenkparallelogramm-Gestänge (18) zugeordnet wird, daß eine entsprechende Gegenkraft durch Gewichtsverlagerung der Flüssigkeit (19) in dem Behälter (15) aufgebaut wird.

9. Stativ nach Anspruch 8, **dadurch gekennzeichnet,** daß der Flüssigkeitsbehälter (15) Mittel zum auswechselbaren Aufsetzen auf das Parallelogramm-Gestänge (18) aufweist und daß er ein in sich abgeschlossenes System darstellt.

10. Stativ nach Anspruch 8, **dadurch gekenn****zeichnet**, daß der Flüssigkeitsbehälter (15) mit einem internen Fluid-Regelkreissystem zur steuerbaren Zu- und Abfuhr von in einem separaten Reservoir befindlichen Flüssigkeit verbunden ist.

## Claims

1. Stand for the mounting of an instrument, preferably an optical observation and/or recording instrument, which is freely positionable relative to at least one reference plane, and including a carrier arm (10), a rotary bearing (9) as well as a pivot arm or a parallelogram linkage (18) with associated force storage element (11), characterised thereby, that a carrier arm (5) is connected by way of the rotary bearing (9) with the carrier arm (10) and borne to be displaceable perpendicularly to the axis of the rotary bearing (9) and the pivot arm or the parallelogram linkage (18) is connected with the carrier arm (10), and that the stand (16) - for the taring and for the reproducible fine positioning of the device, for example a surgical microscope (1), into its respective operative setting - displays means for the displacement of the centre of gravity of the instrument (1), which is attachable to a carrier device (2), inclusive of a possible accessory module into the intersection of a not vertical axis (A-A), which extends parallelly to the carrier arm (5), and a not vertical axis (B-B), which extends through a hinge axis at the lower end of the carrier arm (5), about which the device (1) is tiltable, wherein
- the axes (A-A and B-B) are each perpendicular to the other and
- means are provided for the equalisation of the changing force, which is dependent on the stroke, of the force storage element (11).

2. Stand according to claim 1, characterised thereby, that these means comprise the following components:
(a) a carrier device (2), which consists of at least two subassemblies and to which for example the microscope (1) is fastened on the one hand and which is articulatedly connected with a carrier arm (5) on the other hand, wherein the subassemblies are three-dimensionally displaceable in targeted manner in mutually perpendicular directions by means of a respective adjusting device (3 or 4) for the setting of a stable equilibrium state of the flanged-on microscope (1),
(b) a guide device preferably in the upper region of the carrier arm (5) and consisting of a carriage (6), which is arranged to be displaceable in a guide (7) by means of a spindle (8) along a translational axis (8b) and thereby perpendicularly to the translational directions, which are preset by the adjusting devices (3 or 4), and
(c) a device (13, 14, 20) for the movable suspension or guidance of the force storage element (11) co-operating with the parallelogram linkage (18) for the compensation of the change, which is dependent on stroke or angle, in the force of the force storage element (11) for the purpose of permanent retention of its equilibrium at any height of the adapted instrument (1).

3. Stand according to claim 1 or 2, characterised thereby, that the adjusting devices for the subassemblies each comprise a respective spindle drive (3; 4) and that the adjusting devices (3a; 4a) extend each perpendicularly to the other as well as in one plane.

4. Stand according to at least one of the preceding claims, characterised thereby, that the adjusting devices for the subassemblies or for the carriage (6) extend along non-linear, preferably spherical, paths.

5. Stand according to claim 2, characterised thereby, that the device for the movable mounting of the force storage element (11) comprises a slot (13), which is formed as an elongate hole of circularly arcuate shape, in a plate (20) and in which a spigot (14), which is rigidly connected with the force storage element (11), for example a gas compression spring, is borne free of play as bearing axle.

6. Stand according to claim 5, characterised thereby, that the slot (13) displays a shape which departs from the outline of a circle and is preferably however non-linear.

7. Stand according to at least one of the preceding claims, characterised thereby, that the joints of the parallelogram (18) displays means for the purposefully settable lowering of the joint friction.

8. Stand according to the claims 1 and 2, characterised thereby, that instead of the movable suspension for the compensation of the change, which is dependent on stroke, in the force of the force storage element (11), a container (15) partially filled by a liquid (19) is associated with the parallelogram linkage (18) in such a manner that an appropriate counterforce is built up through redisposition of the weight of the liquid (19) in the container (15).

9. Stand according to claim 8, characterised thereby, that the liquid container (15) displays means for the exchangeable mounting onto the parallelogram linkage (18) and that it represents a self-contained system.

10. Stand according to claim 8, characterised thereby, that the liquid container (15) is connected with an internal fluid regulating loop system for the controllable supply and removal of fluid situated in a separate reservoir.

## Revendications

1. Statif pour le supportage d'un instrument d'observation optique et/ou d'enregistrement, librement positionnable par rapport à au moins un plan de référence, contenant un bras porteur (10), un palier tournant (9) ainsi qu'un bras pivotant ou un parallélogramme articulé (18) à élément accumulateur d'énergie (11) associé, caractérisé en ce qu'un bras porteur (5) est relié par l'intermédiaire du palier tournant (9) au bras porteur (10) et est monté de façon déplaçable perpendiculairement à l'axe du palier tournant (9) et le bras pivotant ou le parallélogramme articulé (18) est relié au bras porteur (10) et en ce que le statif (16) comporte, pour le tarage et pour le positionnement fin reproductible de l'instrument, par exemple d'un microscope chirurgical (1), dans sa position active actuelle, des moyens pour le déplacement du centre de gravité de l'instrument (1) à modules accessoires individuels inclus, qui peut être rapporté sur un dispositif porteur (2), pour amener ledit centre de gravité au point d'intersection d'un axe non vertical (A-A) s'étendant parallèlement au bras porteur (5) avec un axe non vertical (B-B) qui passe par un axe d'articulation à l'extrémité inférieure du bras porteur (5), autour duquel l'instrument (1) est basculable, tandis que
- les axes (A-A) et (B-B) sont perpendiculaires l'un à l'autre et
- des moyens sont prévus pour l'équilibrage de la force changeante de l'élément accumulateur d'énergie (11), dépendante de la course.

2. Statif selon la revendication 1, caractérisé en ce que ces moyens comprennent les éléments de construction suivants:
(a) un dispositif porteur (2) consistant en au moins deux groupes de construction et auquel, d'une part, est fixé par exemple le microscope (1) et lequel, d'autre part, est relié de façon articulée à un bras porteur (5), les groupes de construction étant déplaçables de façon ciblée dans des directions mutuellement orthogonales respectivement au moyen d'un dispositif déplaceur (3 et 4) pour le réglage, dans l'espace, d'un état d'équilibre stable du microscope (1) fixé par bride,
(b) un dispositif de guidage de préférence dans la zone supérieure du bras porteur (5) consistant en un chariot (6) monté de façon déplaçable dans un guidage (7) au moyen d'une tige (8) le long d'un axe de translation (8b) et, ainsi, perpendiculairement aux directions de translation qui sont prédéterminées par les dispositifs déplaceurs (3 et 4), et
(c) un dispositif (13, 14, 20) pour la suspension ou le guidage, variable en position, de l'élément accumulateur d'énergie (11) coopérant avec le parallélogramme articulé (18) pour la compensation de la variation, dépendante de la course et de la position angulaire, de la force de l'élément d'emmagasinage d'énergie (11) dans le but de la conservation permanente de son équilibre à chaque niveau de l'instrument (1) adapté.

3. Statif selon la revendication 1 ou 2, caractérisé en ce que les dispositifs déplaceurs pour les groupes de construction comprennent respectivement une commande à tige (3; 4) et en ce que les dispositifs déplaceurs (3a; 4a) s'étendent orthogonalement l'un à l'autre ainsi que dans un plan.

4. Statif selon au moins l'une des revendications précédentes, caractérisé en ce que les dispositifs déplaceurs pour les groupes de construction ou pour le chariot (6) s'étendent le long de trajectoires non linéaires, de préférence sphériques.

5. Statif selon la revendication 2, caractérisé en ce que le dispositif, pour le supportage, variable en position, de l'élément accumulateur d'énergie (11) comprend une fente (13) réalisée sous forme de trou oblong en forme d'arc de cercle dans une plaque (20), dans laquelle fente est guidé sans jeu un ergot (14) en tant qu'axe de palier rigidement relié à l'élément accumulateur d'énergie (11), par exemple à un ressort de compression à gaz.

6. Statif selon la revendication 5, caractérisé en ce que la fente (13) présente une forme s'écartant du contour d'une circonférence, mais de préférence non-linéaire.

7. Statif selon au moins l'une des revendications précédentes, caractérisé en ce que les articulations du parallélogramme (18) comportent des moyens pour la diminution réglable ciblée du frottement d'articulation.

8. Statif selon les revendications 1 et 2, caractérisé en ce qu'au lieu de la suspension, variable en position, pour la compensation de la variation de force, dépendante de la course, de l'élément accumulateur d'énergie (11), un réservoir (15), rempli partiellement d'un liquide (19), est associé de telle façon aux barres du parallélogramme articulé (18) qu'une force antagoniste correspondante est produite par déplacement de poids du liquide (19) dans le réservoir (15).

9. Statif selon la revendication 8, caractérisé en ce que le réservoir de liquide (15) comporte des moyens pour le montage interchangeable sur la barre de parallélogramme (18) et qu'il représente un système clos en soi.

10. Statif selon la revendication 8, caractérisé en ce que le réservoir de liquide (15) est relié à un système de circuit de réglage à fluide interne pour l'amenée et I'évacuation pilotables d'un liquide se trouvant dans un réservoir séparé.
